# EUROPEAN PATENT APPLICATION

(11) **EP 4 686 701 A1**
(43) Date of publication of application: **04.02.2026**
(21) Application number: 24191395.3
(22) Date of filing: 29.07.2024
(51) Int. Cl.: C01B 32/182, A61K 33/44, A61P 31/00, A61P 31/04, C01B 32/194, G01N 1/28, B82Y 40/00

(54) **MODIFIED GRAPHENE AND MEDICAL USE THEREOF**

(71) Applicant: Univerzita Palackého v Olomouci, 771 47 Olomouc (CZ)
(72) Inventor: Zboril, Radek, Olomouc (CZ); Bakandritsos, Aristides, Olomouc (CZ); Panacek, David, Olomouc (CZ)
(74) Representative: Harber IP s.r.o.

(57) **Abstract**

The present invention provides nitrogen-doped carboxylated graphene with immobilized metal ions, particularly manganese ions. Metal ions with very low antibacterial activity as free ions, after their immobilization on a solid surface as the nitrogen-doped carboxylated graphene, can display potent and persistent antibacterial properties. These novel materials are useful as an antibacterial medicament, in particular for use in the treatment of bacterial and yeast infections in humans and animals.

## Description

### Field of Art

The invention relates to nitrogen-doped carboxylated graphene with immobilized metal ions, particularly manganese ions, and to its use in treatment of microbial infections.

### Background Art

The outbreak of antibiotic-resistant bacteria, or "superbugs", poses a global public health hazard due to their resilience against the most effective last-line antibiotics. Identifying potent antibacterial agents capable of evading bacterial resistance mechanisms represents the ultimate defense strategy. Classical antibacterials include natural or modified proteins and peptides, synthetic small molecules and natural toxins. However, overcoming the problem of bacterial resistance requires opening new fields.

To this end, the development of (nano)materials such as inorganic and carbon-based nanomaterials, metals, polymers, charge- and light-activated nano-systems emerged as an alternative tool for the treatment of infectious diseases, with the potential to circumvent bacterial molecular defense mechanisms. Silver nanoparticles (Ag NPs) remain one of the most potent antibacterial nano-agent known to date, as they are highly effective against a wide range of multidrug-resistant bacteria, exhibiting low minimum concentrations for full-growth inhibition (MIC₁₀₀ of 1-15 mg L⁻¹). Nevertheless, Ag NPs have relatively high non-specific toxicity and it was recently reported that bacteria can develop resistance even to Ag NPs by producing flagellin, a protein that triggers the aggregation of Ag NPs and their deactivation. Such sophisticated defence mechanism could be circumvented either chemically, by adding flagellin molecular inhibitors, blocking flagellin production, or by engineering strong covalent bonds between Ag NPs and the surface of functionalized graphene, thus preventing their aggregation. Despite these breakthrough findings, it is imperative to continue research efforts in order to stay ahead of the emergence of bacterial resistance to flagellin inhibitors, while the use of Ag NPs will remain risky for *in-vivo* application due to their toxicity. The research field of advanced nanomaterials is constantly evolving to address the challenges and limitations, with varying level of success. For instance, a modified MXene exhibiting a MIC value of 200 mg L⁻¹ against *Escherichia coli* and *Staphylococcus aureus,* required light irradiation (generating bactericidal reactive oxygen species), while its cytocompatibility was very similar to its MIC. Nanocubosomes enclosing lipophilic antibacterial peptides (LL-37) with a MIC value of 64 mg L⁻¹ against *Escherichia coli,* also exhibited the same cytocompatibility as the MIC value, and narrow activity spectrum. A reduced MIC value of 25 mg L⁻¹ was achieved with core-shell Pd-Ir nanostructures, however for 60 % inhibition of bacterial growth, and unknown cytocompatibility.

There continues to exist a need for further development of antibacterial agents, having a broad spectrum of activity, effective also against resistant bacteria and with no or low toxicity to healthy human cells. It is crucial that the forthcoming generation of antibacterial agents is designed to circumvent the emergence of bacterial resistance, a phenomenon frequently observed following the recurrent utilization of existing antibiotics.

### Disclosure of the Invention

The present invention provides nitrogen-doped carboxylated graphene (or nitrogen-doped graphene acid, NGA) with immobilized metal ions. The metal ions may preferably be selected from copper ions, iron ions, manganese ions and combinations thereof.

Particularly preferably, the present invention relates to nitrogen-doped carboxylated graphene with immobilized manganese ions. This structure will be abbreviated as NGA-Mn in the following text. The manganese ions are typically manganese(II) ions.

The structure of nitrogen-doped carboxylated graphene with immobilized copper ions will be abbreviated as NGA-Cu, and the structure of nitrogen-doped carboxylated graphene with immobilized iron ions will be abbreviated as NGA-Fe. Generally, the structure of nitrogen-doped carboxylated graphene with immobilized metal ions will be abbreviated as NGA-metal.

NGA-metal is preferably selected from NGA-Mn, NGA-Cu and NGA-Fe.

The terms "nitrogen-doped carboxylated graphene" and "nitrogen-doped graphene acid" are used herein as synonyms. Abbreviation "NGA" is used as an abbreviation for "nitrogen-doped graphene acid".

Nitrogen-doped carboxylated graphene has nitrogen atoms in-plane, and out-of-plane carboxylic groups. Structural characterization shows that in NGA-metal, the metal ions can be coordinated to deprotonated carboxylate groups and protonated carboxylic groups and to nitrogen atoms. In the particularly preferred embodiment of NGA-Mn, the manganese ions can be coordinated to deprotonated carboxylate groups and protonated carboxylic groups and to nitrogen atoms.

In a preferred embodiment, NGA-metal contains 15 to 40 at. % of oxygen, 2 to 10 at. % of nitrogen and 1 to 7 wt. % of metal. Other elements comprise mostly carbon and residual elements (in the method of preparation used in the present invention, the residual element is fluorine).

More preferably, NGA-metal contains 20 to 30 at. % of oxygen, 3 to 7 at. % of nitrogen and 2 to 5 wt. % of metal.

In a particularly preferred embodiment, NGA-Mn contains 15 to 40 at. % of oxygen, 2 to 10 at. % of nitrogen and 1 to 7 wt. % of manganese. Other elements comprise mostly carbon and residual elements (in the method of preparation used in the present invention, the residual element is fluorine).

More preferably, NGA-Mn contains 20 to 30 at. % of oxygen, 3 to 7 at. % of nitrogen and 2 to 5 wt. % of manganese.

The concentration of metal (in a preferred embodiment, of Mn) in the samples was measured with atomic absorption spectroscopy (AAS) using a ContrAA 600 with graphite furnace (Analytik Jena AG, Germany) equipped with a high-resolution Echelle double monochromator (spectral band width, 2 pm at 200 nm) and a xenon lamp as a continuum radiation source. Selected measurements with AAS were cross-checked with an 7500ce inductively coupled plasma mass spectrometer (ICP-MS) instrument (Agilent), with the same results.

The atomic percentages of nitrogen, oxygen, carbon and fluorine were measured with high-resolution X-ray photoelectron spectroscopy (HR-XPS), with a PHI VersaProbe II (Physical Electronics, Japan) spectrometer using an Al Kα source (15 kV, 50 W). The obtained data were evaluated and deconvoluted with the MultiPak (Ulvac - PHI, Inc.) software package. The spectral analysis process involved Shirley background subtraction and peak deconvolution using mixed Gaussian-Lorentzian functions. All binding energies are referenced with respect to C-C bond at 284.8 eV.

HR-TEM images were obtained using a TITAN 60-300 microscope with an X-FEG type emission gun, operating at 300 kV. Scanning transmission electron microscopy high-angle annular dark-field imaging (STEM-HAADF) analysis for EDS (energy-dispersive X-ray spectroscopy) elemental mapping on the products was performed with the same microscope operating at 80 kV.

Preferably, the NGA-metal, in particular the NGA-Mn, is in the form of flakes having the largest dimension between 100 and 200 nm, as determined with scanning electron microscopy (SEM) JEOL 7900F microscope (JEOL, Japan), with accelerating voltage of 5 kV.

The size was also determined with dynamic light scattering, providing a mean hydrodynamic diameter value of 50 to 800 nm, preferably 160 nm. The mean hydrodynamic diameter was measured with Zetasizer Nano ZS instrument (Malvern).

Manganese is an essential bio-element, but it does not show any antibacterial activity on its own. Nitrogen-doped carboxylated graphene (NGA) does not possess any antibacterial activity either. Surprisingly, NGA with immobilized manganese cations shows a potent, wide-spectrum antibacterial activity, including activity against multidrug-resistant bacteria, while maintaining excellent cytocompatibility to human cells. Moreover, NGA-Mn evades bacterial resistance development, even after repeated usage. Preliminary tests showed that NGA-Cu and NGA-Fe also show antibacterial activity.

NGA-metal, in particular NGA-Mn, is endowed with unique features:
(i) it exhibits strong antibacterial activity similar to that of Ag NPs, and other potent molecular antibiotics, even against multidrug-resistant bacterial strains,
(ii) it evades bacterial resistance development, even after 30 serial bacterial generation passages, whereas Ag NPs, for example, develop resistance at the 9^{th} incubation cycle against *Escherichia coli,* and
(iii) it has very high cytocompatibility with healthy human cells (*ca.* 40-fold higher than the MIC₁₀₀), which is essential for considering its further practical application.

NGA-Mn blocks vital bacterial functions related to membrane synthesis *via* multimolecular and bridging binding to biomolecules on the outer parts of the membrane. The current findings reveal a previously untapped and overlooked pathway, which demonstrates how essential micronutrient metal ions can acquire potent antibiotic properties.

The present invention further provides a method for preparation of nitrogen doped carboxylated graphene with immobilized metal ions (NGA-metal), which contains the following steps:
- providing nitrogen doped graphene,
- oxidizing the nitrogen doped graphene, preferably by reaction with an oxidizing inorganic acid, more preferably with nitric acid, to form nitrogen doped graphene acid,
- reacting the nitrogen doped graphene acid with a salt of the metal.

In a particularly preferred embodiment, the present invention provides a method for preparation of nitrogen doped carboxylated graphene with immobilized manganese (NGA-Mn), which contains the following steps:
- providing nitrogen doped graphene,
- oxidizing the nitrogen doped graphene, preferably by reaction with an oxidizing inorganic acid, more preferably with nitric acid, to form nitrogen doped graphene acid,
- reacting the nitrogen doped graphene acid with a manganese(II) salt.

Nitrogen doped graphene is known, e.g., from WO 2021/223783, where it is produced as a supercapacitor material.

Nitrogen-doped graphene may be prepared by the following steps (according to WO 2021/223783):
a) providing a dispersion of fluorinated graphite;
b) subjecting the dispersion of fluorinated graphite to sonication and/or mechanical treatment and/or thermal treatment;
c) contacting the product from step b) with an azide reagent at a temperature of 40 to 200 °C;
d) separating the solid product (nitrogen-doped graphene) formed in step c) from the mixture;
e) optionally dialysis of the product against water.

The term "fluorinated graphite" includes fluorographite, graphite fluoride, fluorinated graphite, and exfoliated forms of these materials. Fluorinated graphites are also available under the name poly(carbon monofluoride), carbon monofluoride or poly(carbon fluoride). The initial content of fluorine in the starting fluorinated graphite is typically at least 40 at. %, more preferably at least 45 or at least 50 at. %, relative to the total atoms present in the sample and determined by X-ray photoelectron spectroscopy (XPS) using an Al-Kα source.

The term "nitrogen doped graphene" means graphene with N-atoms (nitrogen atoms) incorporated in the graphene lattice. This term encompasses single-layer graphene, as well as materials comprising single-layer graphene in a mixture with moieties (e.g., flakes) or particles containing a plurality of graphene layers. However, this term also covers graphene wherein a small proportion (e.g., up to 10 % or up to 5 %) of the nitrogen atoms are bound to the carbon atoms as out-of-plane substituents (e.g. amino-groups), i.e. not incorporated in the graphene lattice. This term also covers graphene wherein a small amount of fluorine is present as well (up to 16.6 at. %; preferably lower than 5 at. %).

Mechanical treatment preferably includes at least one treatment selected from high-shear mixing, stirring, vigorous stirring, stirring with magnetic bar, stirring with a mechanical stirrer.

Thermal treatment preferably includes heating the dispersion in step b) to a temperature within the range of 50 to 250 °C, or from 80 to 200 °C, or more preferably from 100 to 150 °C. It can also include the treatment inside a solvothermal reactor at pressures higher than the normal atmospheric pressure.

The dispersion prepared in step a) is a dispersion of fluorinated graphite in a solvent. The solvent is preferably a polar solvent or a mixture of a polar and a non-polar solvent. The solvent may preferably be selected from dimethylformamide (DMF), dimethylsulfoxide (DMSO), *N*-methyl-2-pyrrolidone (NMP), N,N-dimethylacetamide (DMA), glycols such as ethylene glycol, and mixtures thereof. Less polar or non-polar solvents such as acetonitrile, benzene, toluene or chlorobenzene may be used in combination with a polar organic solvent (for example DMF, NMP, DMSO, DMA).

The step of sonication and/or mechanical treatment and/or thermal treatment yields a mixture containing fluorinated graphene and/or exfoliated fluorinated graphite particles. Sonication is typically carried out at frequency range of 20 kHz to 100 kHz and for a period of at least 2 hours, more preferably of at least 3 hours, even more preferably at least 4 hours. The thermal treatment is typically carried out at the temperature range of 40-200 °C and for a period of at least 1 hour or preferably at least 6 hours, more preferably at least 24 hours, even more preferably 80 hours. The mechanical treatment is most typically carried out by high-shear mixing or magnetic bar stirring.

The azide reagent is preferably added to the solvent of the reaction as a powder or in the form of a suspension in a solvent.

The solvent is preferably a polar solvent. The solvent may preferably be selected from dimethylformamide (DMF), dimethylsulfoxide (DMSO), *N*-methyl-2-pyrrolidone (NMP), *N,N-*dimethylacetamide (DMA), glycols such as ethylene glycol, and mixtures thereof. Less polar or non-polar solvents such as acetonitrile, benzene, toluene or chlorobenzene may be used in combination with a polar organic solvent (for example DMF, NMP, DMSO, DMA). In a particularly preferred embodiment, the solvent is the same as the solvent used for preparing the dispersion of fluorinated graphene prepared in step b).

The azide reagent may preferably be selected from metal azides, tri(C1-C4)alkylsilyl azides. More preferably, the azide reagent is selected from NaN₃, KN₃, LiNs, Pb(N₃)₂, trimethylsilyl azide.

After contacting the product of step b) containing fluorinated graphene with the azide reagent, the mixture is typically subjected to heating to a temperature within the range of 40-200 °C, preferably 70-170 °C, even more preferably 100-140 °C. The heating is preferably carried out for at least 4 hours, preferably for 4 hours to 20 days, even more preferably for at least 8 hours, yet more preferably for at least 24 hours, and even more preferably for at least 2 days (48 hours) or for at least 3 days (72 hours). The longer is the period of heating, the higher is the nitrogen doping.

The step of isolation of the nitrogen-doped graphene may be performed by known techniques such as centrifugation, sedimentation or filtration.

The following general steps refer to the synthesis of NGA from the nitrogen-doped graphene. The step of oxidizing the nitrogen doped graphene by reaction with an oxidizing inorganic acid may preferably be carried out with nitric acid (e.g., 20-98 % (v/v) aqueous nitric acid), more preferably with 40-70 % (v/v) aqueous nitric acid. The reaction is preferably carried out at a temperature within the range of 40-200 °C, preferably 70-170 °C, even more preferably 100-140 °C. The heating is preferably carried out for at least 4 hours, preferably for 4 hours to 20 days, even more preferably for at least 8 hours, yet more preferably for at least 24 hours, and even more preferably for at least 2 days (48 hours).

The nitric acid is preferably added to nitrogen-doped graphene which is in the form of a liquid dispersion.

The resulting nitrogen doped graphene acid may be isolated by any of known techniques such as centrifugation, sedimentation or filtration.

The washing step is carried out in order to purify the nitrogen doped graphene acid. The washing may be performed with hot distilled water (at least 70°C) and distilled water. The purity may be further increased by performing dialysis of the product against water.

The step of reacting nitrogen doped graphene acid (NGA) with metal salt is preferably carried out by mixing aqueous NGA suspension with an aqueous solution of metal salt, sonicating the mixture and mixing the mixture. The dispersion may be further purified by washing and filtration to remove unreacted metal salt.

The metal salt is preferably an inorganic salt of metal, such as an inorganic salt of manganese(II), iron(III) or copper(II). More preferably, the metal salt is a nitrate, more preferably manganese nitrate, iron nitrate or copper nitrate.

The invention further provides the nitrogen doped carboxylated graphene with immobilized metal (NGA-metal) for use as a medicament. More preferably, the invention provides NGA-metal for use an antibacterial medicament, in particular for use in the treatment of bacterial infections in humans and animals.

The bacterial infections are infections caused in particular by bacteria selected from *Enterococcus faecalis. Enterococcus faecium, Staphylococcus aureus, Staphylococcus epidermidis, Escherichia coli, Pseudomonas aeruginosa, Candida albicans, Enterobacter cloacae, Klebsiella pneumoniae, Acinetobacter baumannii.*

The invention further provides a pharmaceutical composition comprising NGA-metal and at least one pharmaceutically acceptable auxiliary compound. Pharmaceutically acceptable auxiliary compounds are known to those skilled in the art of pharmaceutical formulation, and may be selected in particular from solvents, fillers, binders, dispersing agents, flavorants. For example, auxiliary compounds used in the formulations of known antibiotics can be used.

### Brief Description of Drawings

Figure 1. a) XPS survey spectrum and b) HR-XPS of Mn 3s region in NGA-Mn. Owing to the effects of highly electronegative functional groups in the NGA-Mn, the energy splittings are slightly higher than Mn in MnO.
Figure 2. EPR spectra of NGA, NGA with 10 ppm of Mn²⁺ and NGA with 10 ppb of Mn²⁺.
Figure 3. FT-IR spectra of NG, NGA, and NGA-Mn.
Figure 4. Comparison of the whole FTIR spectra of NG, NGA, and NGA-Mn materials.
Figure 5. Comparison of fingerprint regions of NGA FTIR spectra measured from dispersions with pH of 4 and 11 adjusted by addition of NaOH.
Figure 6. (a) XANES, and (b) k2 weighted FT EXAFS in R space for Mn foil, MnO, NGA Mn pure and in presence of *Escherichia coli* (NGA Mn+*E*. *coli*)*.* Wavelet transformation for the k2-weighted EXAFS signal of the (c) Mn metallic foil, (d) MnO, (e) NGA Mn, and (f) NGA Mn+*E*. *Coli.*
Figure 7. DFT calculation showing the preferential coordination site of Mn2+ with NGA (binding distances are shown in Å, and binding energies in kcal mol⁻¹).
Figure 8. HR-TEM images of (a) NG and (b) NGA (inset: SEM image ofNGA). EDS chemical mapping of NGA for (c) carbon, (d) nitrogen, and combined chemical mapping of (e) carbon and nitrogen, (f) carbon and oxygen, (g) HAADF-STEM image of an NGA-Mn flake. EDS chemical mapping of NGA-Mn for (h) oxygen and (i) manganese.
Figure 9. Viability of human lung fibroblasts HEL (n=3), human skin fibroblasts BJ (n=3) treated with NGA Mn and Mn(NOs)z. Error bars represent standard deviations.
Figure 10. *Escherichia coli* and *Staphylococcus aureus* treated for 30 generations (serial passages) with the NGA Mn.
Figure 11. *Escherichia coli* treated for 30 generations with the NGA Mn and for 20 generations with colloidal Ag NPs (mean size 28 nm). Experimental development of resistance is reflected in the changes in relative MIC (defined as the MIC at generation n / MIC at generation 0).

### Examples of carrying out the Invention

Chemicals and Reagents. Graphite, fluorinated polymer > 61 wt. % F - GF, (Millipore Sigma), sodium azide 99% - NaN₃ (Millipore Sigma), dimethylformamide pure -DMF (Lach:ner), absolute ethanol - EtOH (Penta), nitric acid 65% - HNOs (Lach:ner), acetone 99% (Millipore Sigma). Aqueous stock solution of manganese [Mn²⁺] was prepared from the nitrate salt (p.a., Merck). Ultrapure water (18 MΩ cm) was used for all solutions.

**Synthesis of nitrogen doped graphene (NG).** The synthesis of NG was performed according to WO 2021/223783. 10 g of fluorinated graphite was dispersed in a glass flask in 300 ml of DMF and stirred for 72 hours, then sonicated for 4 hours. Then 30 g of NaN₃ was added to the previous mixture transferred to spherical flask and left stirring and heating to 130°C for 3 days with a condenser in the hood. After the end of the reaction, the sample was washed in falcons with DMF (3x), acetone (3x), ethanol (3x), distilled water (3x) and hot distilled water (2x), using centrifugation (14000 rcf).

**Synthesis of nitrogen-doped carboxylated graphene (nitrogen-doped graphene acid, NGA).** The synthesis of NGA was performed according to PCT/CZ2022/050042, using 45 % (w/w) of HNO₃ instead of 65 %. An amount of the previously prepared N-doped graphene derivative was treated with 45% (w/w) HNOs for 48 hours at 130°C in a glass flask with the condenser. After completion of the reaction, the product was purified by washing with filtration (Whatman cellulose membrane filter, pore size 0.2 µm); 5 times with hot distilled water, 5 times with distilled water and purified by dialysis (dialysis tubing cellulose membrane, 14 kDa cutoff).

For antibacterial applications, after the purification step, the NGA was filtered using a 200 nm cellulose filter in the first step, then the filtrate was taken and filtered again using a 100 nm cellulose filter (Millipore cellulose membrane filter, pore size 0.1 µm). The NGA remaining on the 100 nm filter was used to immobilize the manganese ions.

**Synthesis of NGA-Mn.** For this purpose, a 5 mL aqueous suspension containing 25 mg of NGA was mixed with 10 mL of 30×10⁻³ M Mn(NO₃)₂ solution and sonicated for 30 min, Then the mixture was stirred vigorously for 24 h at room temperature, . The dispersion of the NGA with immobilized Mn²⁺ was purified by washing five times with distilled water on a filtrater paper (Millipore cellulose membrane filter, pore size 0.1 µm) in order to remove the manganese ions not firmly coordinated on the NGA flakes.

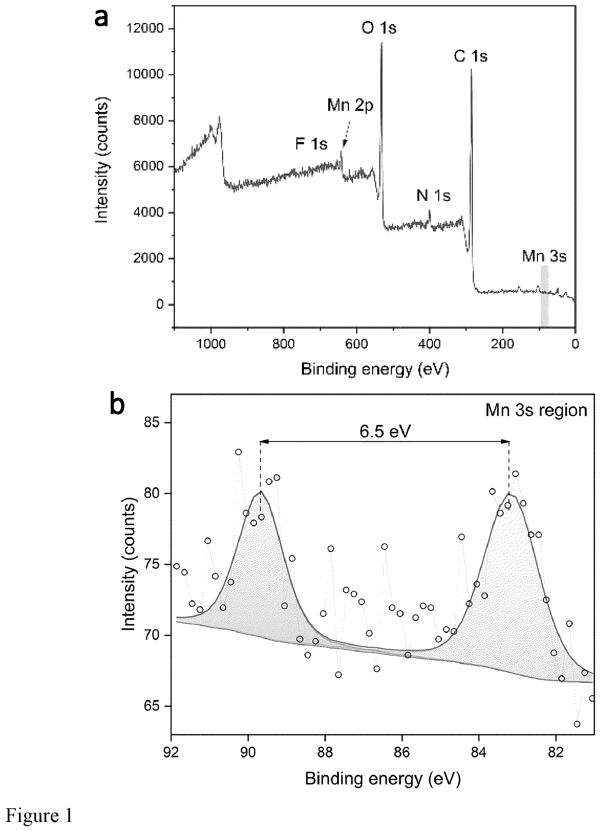

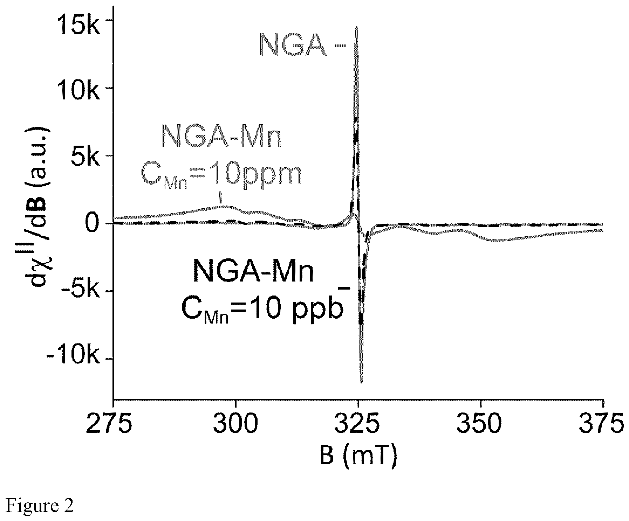

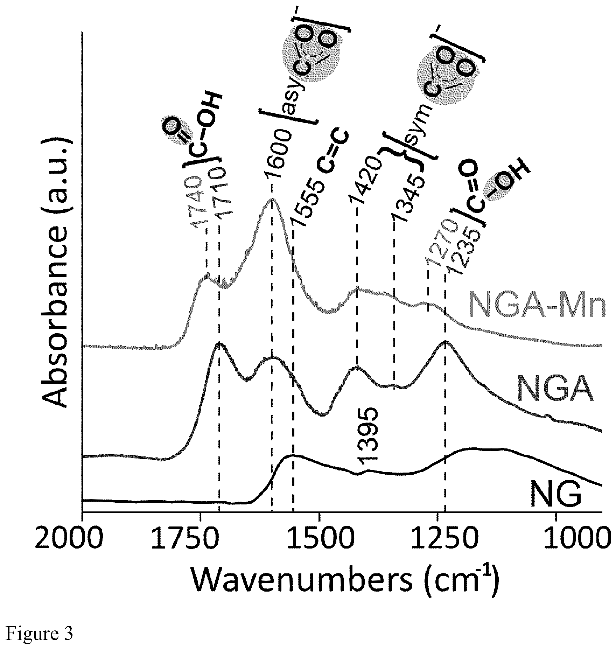

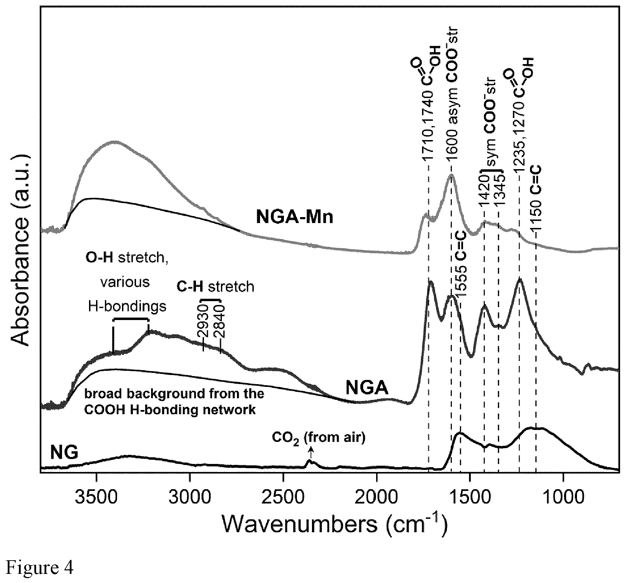

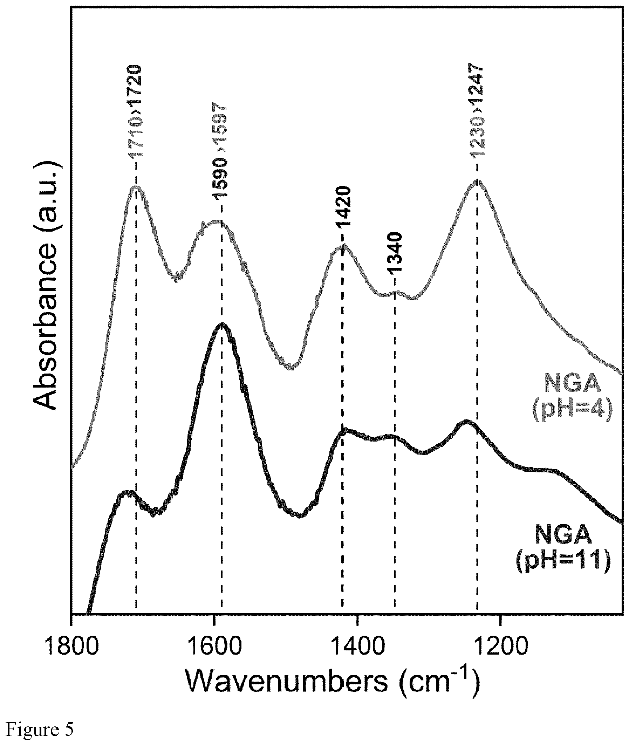

Immobilization of Mn²⁺ on NGA resulted in a manganese loading of 4.5 wt.% according to atomic absorption spectroscopy (AAS) and according to inductively coupled plasma mass spectrometry. The oxidation state of Mn²⁺ after coordination remained the same, as determined by the energy difference in the multiplet splitting in the 3s region by 6.5 eV, according to the XPS results (Figure 1a,b). The preservation of the Mn(II) characteristics was also confirmed by EPR (Figure 2). The carboxylic groups that were detected by FTIR were partially ionized to carboxylates after the interactions with Mn²⁺, as indicated by the emergence of the vibrations at 1600 cm⁻¹ and at 1420/1345 cm⁻¹ in NGA-Mn (Figure 3-5).

We further probed the NGA-Mn interactions with electron paramagnetic resonance spectroscopy (EPR), since both Mn²⁺ and NGA are spin-active. NGA in water (*T* =80 K) (Figure 2) shows isotropic and narrow derivative resonance signal (ΔBₚₚ = 0.9 mT), centred at *g*ᵢₛₒ=1.998, which is associated to S=1/2 spin centres that are localized onto the aromatic-rich regions of the carbon framework (type C-sp², =C^{•}-) and to spin containing defects placed near/on the carboxyl groups (O=C^{•}-). The Mn(NO₃)₂ salt dissolved in water displays broad and featureless resonance signal, with a peak-to-peak line width of about 77.6 mT at g=2.018. Upon addition of Mn²⁺ ([Ar] 3d⁵) at 10 ppm and 10 ppb to the NGA-water dispersion a clear decrease of the NGA's radical signal intensity is observed; the phenomenon results from the electronic interaction, and thus binding of the Mn²⁺ ions with the NGA scaffold. The estimated ⁵⁵Mn nuclear hyperfine coupling from EPR simulation (Aᵢₛₒ = 9.8 mT) is in harmony with those observed in hexacoordinate Mn²⁺ enzymes, containing a set of N, O donors in distorted octahedral geometries.

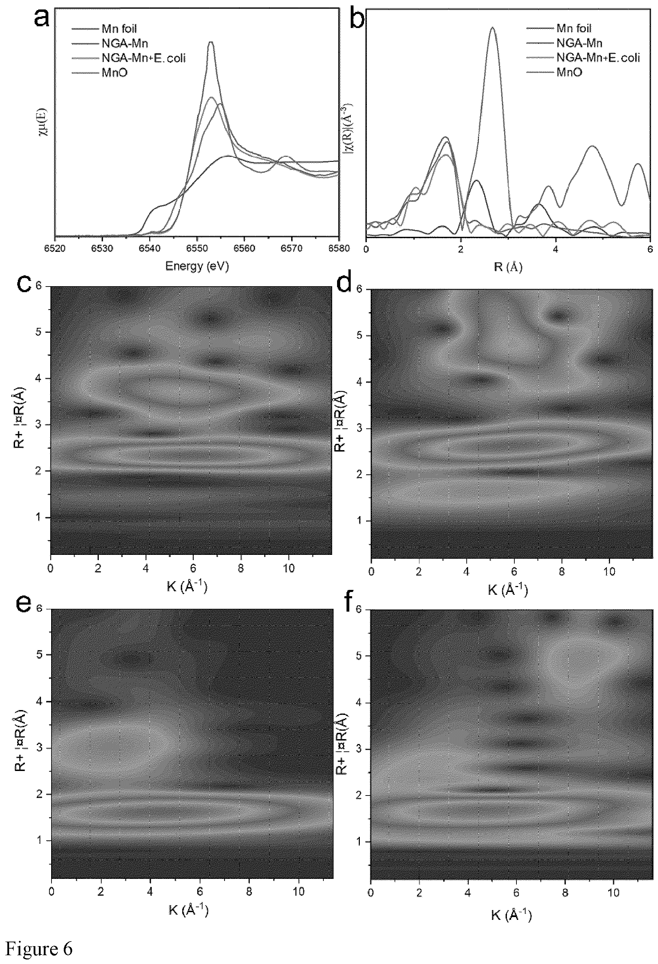

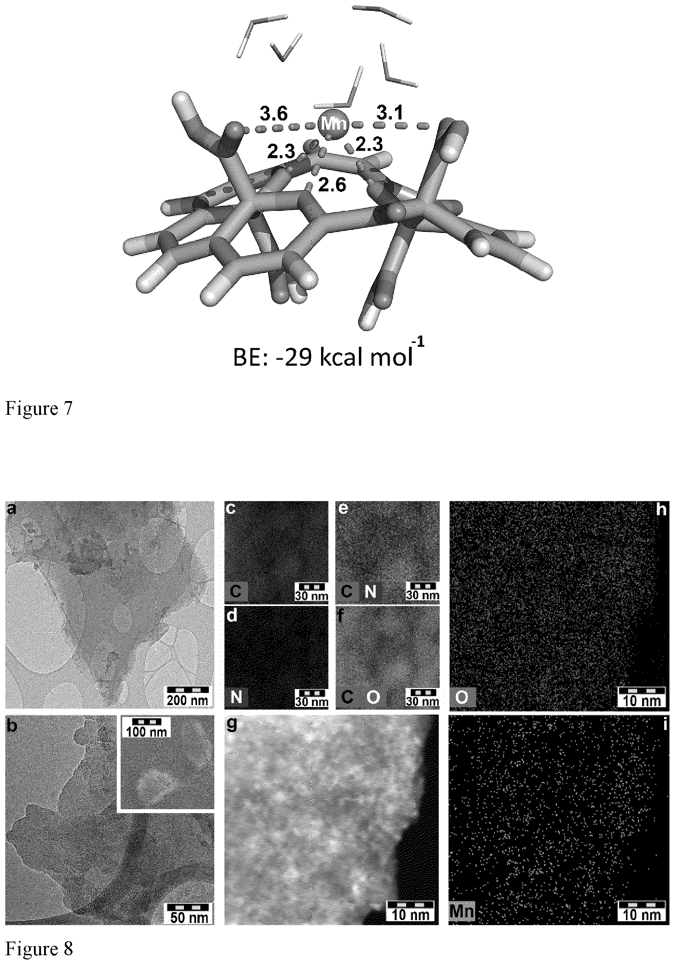

The coordination of Mn²⁺ cations with light atoms (nitrogen and oxygen) from NGA's functionalities and their single atomic state was verified due to the absence of scattering at radial distances above 2 Å (corresponding to metallic Mn-Mn or oxide Mn-O-Mn bonds), as evidenced by the extended X-ray absorption near edge (XANES) and extended X-ray absorption fine structure spectroscopy results (Figure 6). In agreement to these observations, density functional theory (DFT) calculations revealed the coordination bonds between Mn²⁺, the -COOH groups and nitrogen dopants in NGA according to the natural bond orbital analysis (Figure 7). The role of N-doped vacancies in the coordination of Mn²⁺ was also indicated by the second-order perturbation analysis estimating the strength of donor-acceptor interactions, showing a stronger donation to the vacant orbitals of the metal ions from N compared to the carboxylic oxygens.

The analysis of NG, NGA, and NGA-Mn by high resolution transmission electron microscopy (HR-TEM) showed that NG comprised few-layered graphene flakes with size around 600 nm (Figure 8 a). The NGA flakes were smaller, *ca.* 150 nm, due to the oxidative cutting during its synthesis according to HR-TEM (Figure 8 b) and scanning electron microscopy (SEM) (inset in Figure 8 b). Elemental chemical mapping of NGA confirmed the homogeneous coverage of the flakes with nitrogen and oxygen, coinciding with the spatial distribution of carbon (Figure 8 c-h). After the interaction of NGA with Mn²⁺ ions no Mn-based nanoparticles were observed on the NGA surface, as confirmed by high-angle annular dark-field scanning transmission electron microscopy (HAADF-STEM), (Figure 8 g), corroborating the EXAFS results. Elemental chemical mapping revealed a homogeneous coverage of graphene flakes with Mn cations, as well as oxygen atoms from the carboxyl groups of NGA (Figure 8 i).

**Table 1. Atomic contents of individual elements in NG, NGA, NGA-Mn according to HR-XPS surface analysis are as following:**

| | Element (in atomic %) | | | |
|---|---|---|---|---|
| | C | O | N | Mn |
| N-doped graphene | 81.6 | 3.3 | 12.7 | - |
| N-doped graphene acid | 69.0 | 24.7 | 5.2 | - |
| NGA-Mn | 60.8 | 27.6 | 4.4 | 2.5 |

**Synthesis of NGA-Fe, and NGA-Cu.** The same procedure (as in the case of NGA-Mn as previously described) was followed for immobilizing Fe or Cu cations, using Fe(NO₃)₃ or Cu(NO₃)₂, respectively, as metal ion precursors to prepare the respective graphene derivatives NGA-Fe, and NGA-Cu. The metal loading for the NGA-Fe, and NGA-Cu products was 4.1 and 4.4 wt.%, respectively, according to AAS and to ICP.

**Synthesis of colloidal silver nanoparticles (comparative antibacterial material).** Colloidal silver 28 nm sized particles were synthesized *via* a modified Tollen's method using maltose as reducing agent, according to Panáček, A. *et al.* ("Bacterial resistance to silver nanoparticles and how to overcome it,, *Nat. Nanotechnol.* **13,** 65-71 (2018). Briefly, [Ag(NH₃)²]⁺ complex (prepared by mixing AgNO₃ with NH₄OH solution) was reacted with maltose at room temperature for 5 min. The initial concentrations of the reaction components were 10⁻³ mol L⁻¹ and 0.01 mol L⁻¹ for AgNO₃ and the reducing agent, respectively. The concentration of ammonia was 0.005 mol L⁻¹.

**Antibacterial assay.** Antibacterial activity was evaluated by determining MIC (minimum inhibitory concentration) and MBC (minimum bactericidal concentration) using the standard microdilution method according EUCAST (The European Committee on Antimicrobial Susceptibility Testing - EUCAST. http://www.eucast.org). Tested substances were diluted in MH medium (Mueller Hinton broth (MH broth), BIORAD, ref. 69444: Dehydrated beef extract infusion 2 g L⁻¹, Casein hydrolyzate 17.5 g L⁻¹, Starch 1.5 g L⁻¹. Final pH: 7.3 ± 0.1 Ca²⁺ 20-25 mg L⁻¹ Mg²⁺ 10-12.5 mg L⁻¹) to obtain a concentration range between 256 mg L⁻¹ and 2 mg L⁻¹. The plates were inoculated with a standard amount of the tested microbe - the initial density of the inoculum was equal to 5 × 10⁵ CFU/ml. MICs were read after 18 ± 2 hours of incubation at 35 ± 1°C. The same procedure with slight modifications was used to determine MIC of yeast tested. The test substances were diluted in Sabouraud broth (BIORAD, Peptone 11 g L⁻¹, glucose 20 g L⁻¹, Final pH 6.0 - 6.3) with 2% glucose (BioRad, France). MICs were read after 48 h of incubation at 35 ± 1 °C. The MIC was defined as the lowest concentration of antibacterial with no visible growth. To determine MBC, 10 µl from each well with visibly inhibited growth was inoculated onto MH agar/Sabouraud agar (Trios, Czech Republic) and incubated for another 18 h at 35±1 °C. The MBC is defined as the lowest drug concentration which kills 99.9% of the test microorganisms in the original inoculum. All tests were performed in duplicate.

**Bacterial Strains.** Standard reference strains *Enterococcus faecalis* CCM 4224, *Staphylococcus aureus* CCM 4223, *Staphylococcus epidermidis* CCM 7221, *Escherichia* coli CCM 3954, *Pseudomonas aeruginosa* CCM 3955 and *Candida albicans* CCM 8161 from the Czech Collection of Microorganisms (CCM, Brno, Czech Republic) were tested. Bacterial strains with confirmed antibiotic resistance were also included in the experiment. Methicillin-resistant *Staphylococcus aureus* 4591/A (PBP2a+), vancomycin-resistant *Enterococcus faecium* 419/ANA (VanA), multidrug-resistant *Escherichia coli* CE5556 (CTX-M-15, gyrA, aminoglycoside resistant) and multidrug-resistant *Pseudomonas aeruginosa* (PDC+) strains were obtained from the culture collection of Department of Microbiology, Faculty of Medicine and Dentistry, Palacky University Olomouc, Czech Republic. Strains with confirmed extended-spectrum beta-lactamase or carbapenemase production were obtained from NCTC (UK): *Escherichia coli* NCTC 13353 (CTX-M-15), *Enterobacter cloacae* NCTC 13406 (AmpC beta-lactamase), *Klebsiella pneumoniae* NCTC 13438 (KPC-3 carbapenemase), *Klebsiella pneumoniae* NCTC 13443 (NDM-1), *Acinetobacter baumannii* NCTC 13301 (OXA-23, OXA-51-like). *Escherichia coli* NCTC 13846 was colistin resistant (mcr-1). All tested microorganisms were identified by the MALDI-TOF Biotyper system (Bruker Daltonics, Germany) and stored in cryotubes (ITEST plus, Czech Republic) at -80 °C.

**Table 2. MIC (mg L⁻¹) values ofNGA-Mn and comparative compounds (according to literature) tested against the ESKAPE pathogens. Also, the antibacterial activity ofNGA-Fe and NGA-Cu is shown for Escherichia coli:**

| | Bacterial strain | Macolacin^{a} | ETX0462^{b} | Cilagicin^{c} | NGA-Mn | NGA-Fe | NGA-Cu |
|---|---|---|---|---|---|---|---|
| *E* | *Enterococcus faecium- resistant* | >125 | ineffective | 1 | 16 | - | - |
| *S* | *Staphylococcus aureus* - *resistant* | >125 | ineffective | 1 | 16 | - | - |
| *K* | *Klebsiella pneumoniae* | 1 | 4 (MIC₉₀) | >64 | 16 | - | - |
| *A* | *Acinetobacter baumannii* | 1 | 4 (MIC₉₀) | 8 | 4 | - | - |
| *P* | *Pseudomonas aeruginosa* | 4 | 4 | >64 | 4 | - | - |
| *E* | *Enterobacter cloacae* | 4 | NA | >64 | 4 | - | - |
| | *Escherichia coli* | - | - | - | 4 | 56 | 56 |

| | | | | | | | |
|---|---|---|---|---|---|---|---|
| ^{a} Wang, Z. et al. Nature 601, 606-611 (2022). ^{b} Durand-Reville, T. F. et al. Nature 597, 698-702 (2021). ^{c} Wang, Z. et al. Science 376, 991-996 (2022). | | | | | | | |

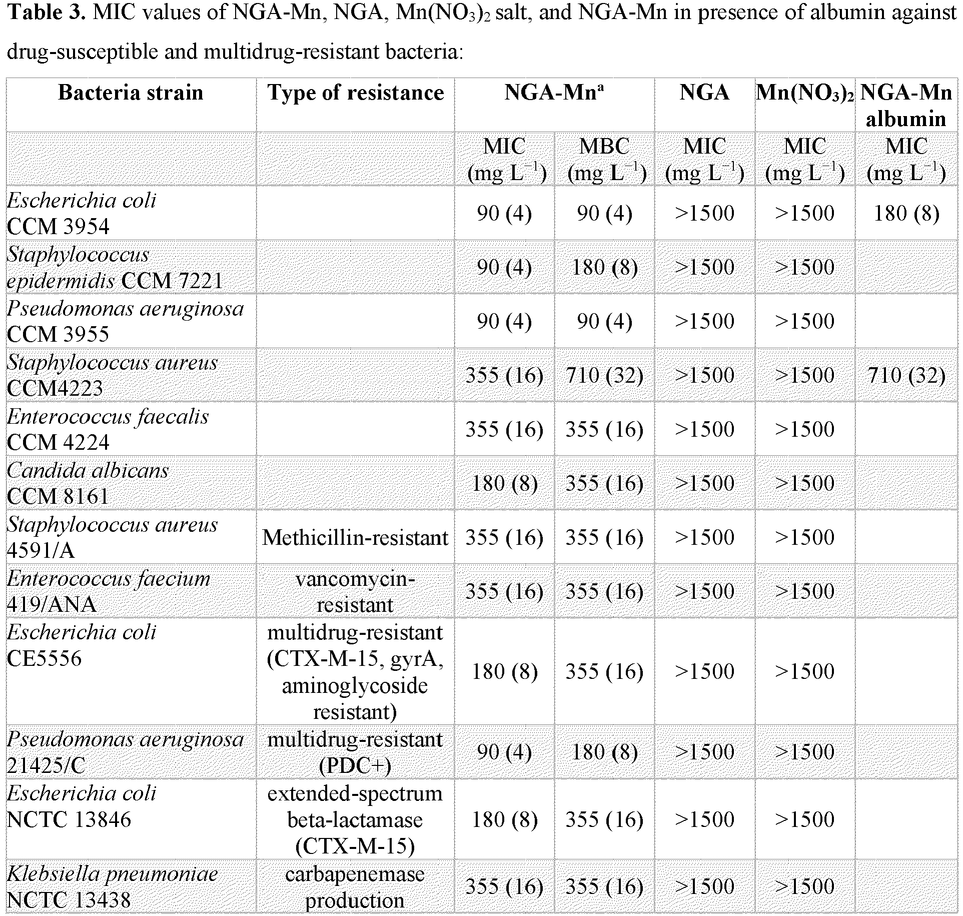

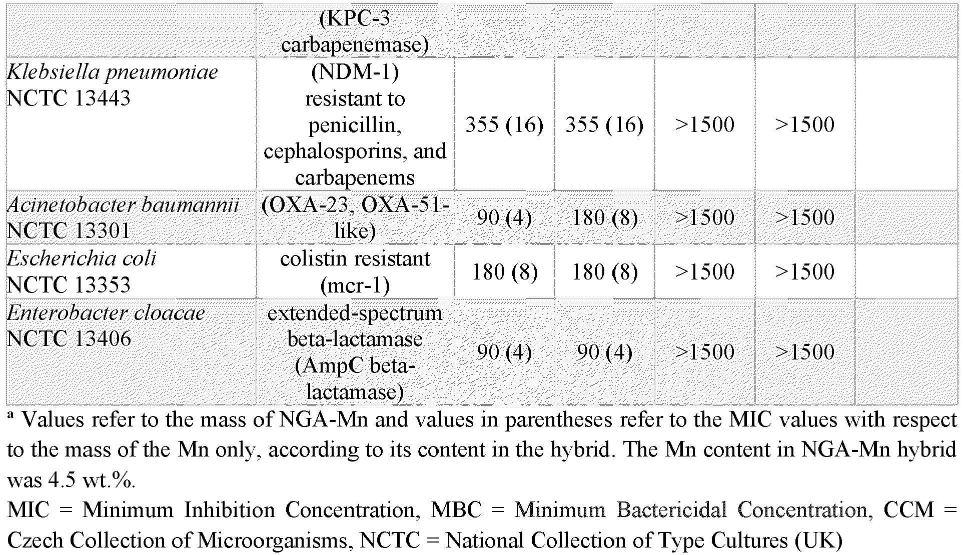

**Cell cultures and cytotoxicity/cell viability assay.** For cellular toxicity, human lung fibroblasts HEL 12469 and human skin fibroblasts BJ (ATCC) cells were cultivated at 37 °C under a 5% CO₂ atmosphere in EMEM-Eagle's Minimum Essential Medium (Sigma-Aldrich), enriched by L-Glutamine, non-essential amino acids (NEAA), fetal bovine serum (FBS), PenStrep (5000 U penicillin, 5 mg streptomycin mL⁻¹), and sodium bicarbonate (7.5%). The HeLa cells were cultivated at 37 °C under a 5% CO₂ atmosphere in DMEM - Dulbecco's modified Eagle's medium (Invitrogen, USA) supplemented with L-Glutamine, 10% FBS, and 1% PenStrep (10000 U penicillin, 10 mg streptomycin mL⁻¹).

Cell viability was evaluated using a BD FACS Verse flow cytometer (BD Biosciences, USA). Ten thousand cells were seeded per well in a 96-well plate. Cells were incubated with NGA-Mn at various concentrations (based on the total mass) from 8 to 120 mg L⁻¹ for 24 h. After 24 h, the supernatant was collected, and cells were gently washed with PBS solution (0.1 M, 7.4 pH). Then, cells were detached with trypsin (0.25% in EDTA, Sigma-Aldrich), resuspended in 100 µL of culture media and added to supernatant. Viability of cells was determined by propidium iodide (PI) and calcein-AM fluorescent probes. Cells were incubated with 1 µL of PI (1 µg mL⁻¹) and 2 µL of calcein-AM, diluted in DMSO (50 µM), for 20 minutes and the fluorescent signal was measured by flow cytometer using red channel (exc. 488/em. 586) for PI and green channel (exc. 488/em. 527) for calcein. Red signal of PI revealed dead cells, which lost their membrane integrity, while green signal was represented by cells with active intracellular esterases that catalysed the non-fluorescent calcein-AM to highly fluorescent green calcein. The viability was established and normalized to control cells with 100% viability.

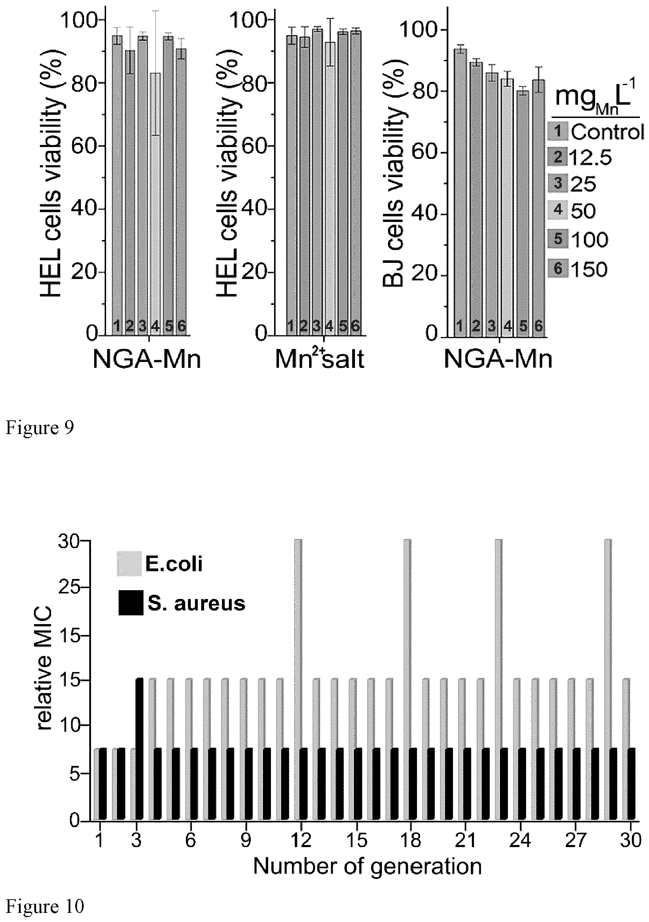

An equally important aspect of antibacterial agents is their biocompatibility. The cytocompatibility of NGA-Mn was investigated with flow cytometry (using propidium iodide and calcein fluorescent probes, see "Methods") on human skin fibroblasts, because of the potential application of antibacterial agents on skin, and on human lung fibroblasts (HEL 12469) for further establishment of the cytocompatibility profile. NGA-Mn was fully tolerated by both cell lines up to 3300 mg L⁻¹ (or 150 mg_{Mn} L⁻¹, Figure 9) with more than 80% cell viability, which is *ca.* 10- to 40-fold higher (depending on the bacterial strain) than its antibacterial MIC values (Table 2-4). The Mn²⁺ salt exhibited the same cytocompatibility values as NGA and NGA-Mn (Figure 9). Manganese leaching tests from the NGA-Mn confirmed the experiments and simulations about the strong binding of Mn, since after 72 h of shaking in water or in cell-culture medium, Mn leaching reached 0.15 mg L⁻¹, well below the toxic levels of NGA-Mn or Mn²⁺ salt (150 mg_{Mn} L⁻¹, Figure 9). The leached amount of Mn corresponded to 0.05 % of the total amount of Mn originally contained in NGA-Mn (300 mg L⁻¹) that was added to the leaching test solution. These results underscore the application potential of the NGA-Mn antibiotic, particularly considering the general cytotoxic effect of silver-based agents or the yet unknown cytocompatibility of other state-of-the-art SAE antibacterials. Such high cytocompatibility, combined with the potent and persistent antibacterial activity of NGA-Mn against multidrug-resistant bacteria may provide a new thrust in the battle against superbugs.

**Induction of resistance.** Induction of bacterial resistance was performed by the dilution micromethod by repeated exposure of *Escherichia coli* CCM 3954 and *Staphylococcus aureus* CCM 4223 strains to sub-inhibitory concentrations of the test compounds of AgNPs and NGA-Mn. The test substances were exponentially diluted in MH culture broth (Mueller Hinton Broth, Biorad, France; the highest concentration was 128 mg L⁻¹. The prepared plates were stored at -20°C.

Bacterial cultures were prepared from inoculum in saline and inoculated into a pre-prepared microtiter plate. The initial concentration of the bacterial suspension in the well was approximately 5×10⁵ CFU/ml. After 18 hours of incubation at 35 °C, the MIC value was read. After incubation and MIC reading, 10 µl of the bacterial suspension from the well with the highest sub-inhibitory concentration of the test substance was plated onto blood agar and incubated for 24 hours at 35 °C. The bacterial culture thus obtained was used for the next cycle. The procedure described represents 1 cycle of resistance induction and a total of 30 cycles were performed. After the 10th, 16th, and 30th cycles, the MICs of the original strains were determined and compared with the MICs of the strains after induction.

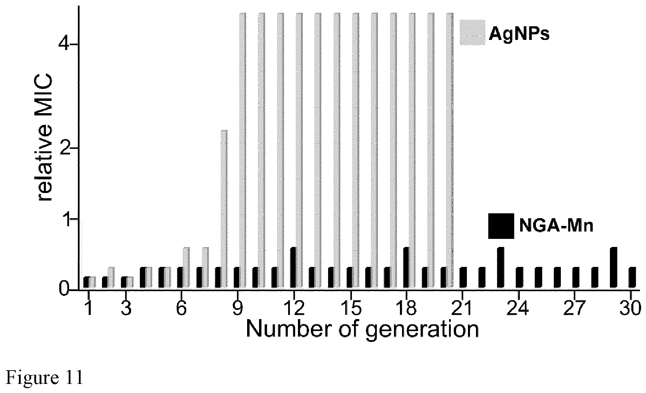

The MIC₁₀₀ of NGA-Mn increased marginally in the case of *Escherichia coli,* and no changes were observed for *Staphylococcus aureus* (Figure 10). An eightfold increase in the MIC value is considered indicative of resistance development. The absence of resistance development suggests that the NGA-Mn's target is not a protein. For comparison, after exposing *Escherichia coli* to Ag NPs resistance was developed at the 9^{th} cycle, indicated by the twenty-eight-fold increase in MIC (Figure 11). These findings predispose NGA-Mn as a next generation, persistent and broad-spectrum antibacterial agent, with a combination of features that are challenging to achieve otherwise. Moreover, the high antibacterial activity ofNGA-Mn against multidrug-resistant bacteria substantially outperforms recently reported inorganic systems, since NGA-Mn exhibits lower MIC values and does not require light irradiation, ultrasonic action or the co-presence of additional agents to generate reactive oxygen species.

**Table 4. MIC values of the NGA-Mn compared with state-of-the-art antibacterial agents:**

| **Antibacteria l agent** | **Incubation time [h]** | **MIC^{a} [mg L⁻¹]** | | | | **Cytocompatibility for human cells^{b} [mg L⁻¹]** | **Resistance (cycles)** |
|---|---|---|---|---|---|---|---|
| | | ***E. coli*** | ***S*. *aureus*** | ***K. pneumoniae*** | ***A*. *baumannii*** | | |
| **NGA-Mn** | 18 | 90^{c} 100% | 355^{c} 100% | 355^{c} 100% | 90^{c} 100% | 2760/3450 100% / 80% | 30 |
| **Bi2S3/Ti3C2 Tx MXene** | 0.17 | 200 100% | 200 100% | NA | NA | 200 100% | NA |
| **GMO-LL-37** | 18 | 64 ∼85% | | NA | NA | 64 80% | NA |
| **Guanidine Nanogels** | 11 | NA | 120 90% | NA | NA | 120 100% | NA |
| **Si sheets** | NA | 200 100% | 100% | NA | NA | NA | NA |
| **CaO₂** | 18 | 100 100% | NA | NA | NA | NA | NA |
| **Pd@Ir** | 12 | 25 ∼60% | NA | NA | NA | NA | NA |

| **Antibacteria l agent** | **Incubation time [h]** | **MIC^{a} [mg L⁻¹]** | | | | **Cytocompatibility for human cells^{b} [mg L⁻¹]** | **Resistance (cycles)** |
|---|---|---|---|---|---|---|---|
| | | ***E. coli*** | ***aureus*** | ***S. K. A. pneumoniae*** | ***baumannii*** | | |
| **NGA-Mn** | 18 | 4^{d} 100% | 16^{d} 100% | 16^{d} 100% | 4^{d} 100% | 100/150 100% / 80% | 30 |
| **macolacin** | 16 | NA | inactive | 1 100% | 1 100% | 2 100% | NA |
| **synthetic molecule ETX0462** | 36 | 1 90% | inactive | 4 90% | 4 90% | NA | NA |
| **arylomycin peptides** | NA | 0.13 100% | 0.06 100% | 0.13 100% | 1 100% | 41 100% | 1 |
| **teixobactin** | 18 | 25 | 0.25 | >40 | NA | 100 100% | 27 |

| | | | | | | | |
|---|---|---|---|---|---|---|---|
| ^{a,b}The percentiles appearing bellow the MIC values refer to cases where the bacterial growth inhibition was not 100%, or where cytocompatibility did not refer to 100% viability of the cells. "NA" stands for unavailability of data. ^{C}For the comparisons with antibacterial nanomaterials the MIC values with respect to the full mass of NGA-Mn was used in order to allow comparisons with the inorganic/nanostructured materials. ^{d} MIC values for the NGA-Mn expressed with respect to the concentration of Mn²⁺ in order to allow comparisons with the molecular antibiotics that do not contain any carrier/matrix. Sources of the data for comparative compounds: Bi₂S₃/Ti₃C₂Tx MXene - Li, J. et al. Nat. Commun. 12, 1224 (2021); GMO-LL-37 - Zabara, M. et al. Adv. Funct. Mater. 29, 1904007 (2019); Guanidine Nanogels - Han, H. et al. Adv. Funct. Mater. 29, 1806594 (2019); Si sheets - Luo, Y. et al. Adv. Healthc. Mater. 9, 1901375 (2020); CaO₂ - Shen, S. et al. Small 15, 1902118 (2019); Pd@Ir - Cai, T. et al. ACS Nano 13, 12694-12702 (2019); macolacin - Wang, Z. et al. Nature 601, 606-611 (2022); ETX0462 - Durand-Reville, T. F. etal. Nature 597, 698-702 (2021); arylomycinpeptides - Smith, P. A. et al. Nature 561, 189-194 (2018); teixobactin - Shukla, R. et al. Nature 608, 390-396 (2022). | | | | | | | |

## Claims

1. Nitrogen-doped carboxylated graphene with immobilized metal ions.

2. Nitrogen doped carboxylated graphene with immobilized metal ions according to claim 1, wherein the metal ions are manganese ions, copper ions or iron ions.

3. Nitrogen-doped carboxylated graphene with immobilized metal ions according to claim 1 or 2, wherein the metal ions are coordinated to deprotonated carboxylate groups and/or protonated carboxylic groups and/or to nitrogen atoms.

4. Nitrogen-doped carboxylated graphene with immobilized metal ions according to any one of claims 1 to 3, which contains 15 to 40 at. % of oxygen, 2 to 10 at. % of nitrogen and 1 to 7 wt. % of the metal, as determined by high-resolution X-ray photoelectron spectroscopy analysis for oxygen and nitrogen and by atomic absorption spectroscopy (AAS) for the metal.

5. Nitrogen-doped carboxylated graphene with immobilized metal ions according to any one of claims 1 to 3, which contains 20 to 30 at. % of oxygen, 3 to 7 at. % of nitrogen and 2 to 5 wt. % of the metal, as determined by high-resolution X-ray photoelectron spectroscopy analysis for oxygen and nitrogen and by atomic absorption spectroscopy (AAS) for the metal.

6. Nitrogen-doped carboxylated graphene with immobilized metal ions according to any one of claims 1 to 5, which is in the form of flakes having the largest dimension of between 100 and 200 nm, as determined by scanning electron microscopy.

7. Nitrogen-doped carboxylated graphene with immobilized metal ions according to any one of claims 1 to 5 which is in the form of flakes having 50 to 800 nm of hydrodynamic diameter as determined by dynamic light scattering measurement.

8. A method for preparation of nitrogen doped carboxylated graphene with immobilized metal ions according to any one of claims 1 to 7, said method containing the following steps:
- providing nitrogen doped graphene,
- oxidizing the nitrogen doped graphene, preferably by reaction with an oxidizing inorganic acid, more preferably with nitric acid, to form nitrogen doped carboxylated graphene,
- reacting the nitrogen doped carboxylated graphene with a salt of the metal.

9. The method according to claim 8, wherein the step of reacting nitrogen doped carboxylated graphene with metal salt is carried out by mixing aqueous or non-aqueous nitrogen doped carboxylated graphene suspension with a solution of the metal salt, isolating the solid, and preferably followed by a further purification of the resulting solid by washing and filtration to remove unreacted metal salt.

10. Nitrogen-doped carboxylated graphene with immobilized metal ions according to any one of claims 1 to 7 for use as a medicament.

11. Nitrogen-doped carboxylated graphene with immobilized metal ions according to any one of claims 1 to 7 for use as an antibacterial medicament.

12. Nitrogen-doped carboxylated graphene with immobilized metal ions according to any one of claims 1 to 7 for use in the treatment of bacterial and yeast infections in humans and animals.

13. Nitrogen-doped carboxylated graphene with immobilized metal ions according to any one of claims 1 to 7 for use in the treatment of bacterial and/or yeast infections caused by bacteria selected from *Enterococcus faecalis. Enterococcus faecium, Staphylococcus aureus, Staphylococcus epidermidis, Escherichia coli, Pseudomonas aeruginosa, Candida albicans, Enterobacter cloacae, Klebsiella pneumoniae,* and *Acinetobacter baumannii.*

14. A pharmaceutical composition comprising nitrogen-doped carboxylated graphene with immobilized metal ions according to any one of claims 1 to 7 and at least one pharmaceutically acceptable auxiliary compound, preferably selected from solvents, fillers, binders, dispersing agents, flavorants and/or molecular antibiotics.
